# EUROPEAN PATENT SPECIFICATION

(11) **EP 4 528 630 B1**
(45) Date of publication and mention of the grant of the patent: **22.04.2026**
(21) Application number: 23198691.0
(22) Date of filing: 21.09.2023
(51) Int. Cl.: G06T 3/4038, A61B 6/46, A61B 6/02, A61B 6/51

(54) **METHOD OF GENERATING A FINAL PANORAMIC IMAGE USING A DIGITAL 3D MODEL OF THE DENTAL CONDITION OF A PATIENT**
VERFAHREN ZUR ERZEUGUNG EINES PANORAMISCHEN ENDBILDES UNTER VERWENDUNG EINES DIGITALEN 3D-MODELLS DES ZAHNZUSTANDS EINES PATIENTEN
PROCÉDÉ DE GÉNÉRATION D'UNE IMAGE PANORAMIQUE FINALE À L'AIDE D'UN MODÈLE 3D NUMÉRIQUE DE L'ÉTAT DENTAIRE D'UN PATIENT

(43) Date of publication of application: 26.03.2025
(73) Proprietor: Sirona Dental Systems GmbH, 64625 Bensheim (DE); Dentsply Sirona Inc., York, PA 17401 (US)
(72) Inventor: Özer, Dr., Alpdeniz, 64625 Bensheim (DE); Eichner, Stefan, 64625 Bensheim (DE)
(74) Representative: Aldridge, Henry Alexander

(56) References cited:
- KR-B1- 101 146 862
- US-A1- 2020 234 404
- BARONE SANDRO ET AL: "Geometrical modeling of complete dental shapes by using panoramic X-ray, digital mouth data and anatomical templates", COMPUTERIZED MEDICAL IMAGING AND GRAPHICS, PERGAMON PRESS, NEW YORK, NY, US, vol. 43, 20 January 2015 (2015-01-20), pages 112 - 121, XP029244417, ISSN: 0895-6111, DOI: 10.1016/J.COMPMEDIMAG.2015.01.005

## Description

### TECHNICAL FIELD OF THE INVENTION

The present invention relates to a computer-assisted x-ray system and a method of generating a final panoramic image of the dental condition of a patient.

### BACKGROUND ART OF THE INVENTION

Panoramic images give dentists a quick overview of a patient's dental condition. Panoramic images are used by dentists to prepare for treatment and allow a quick initial diagnosis in problem cases. A panoramic image is a standard procedure of all extraoral dental radiographic procedures. A classic planar 2D representation of the panoramic image is commonly used by dentists. The panoramic image is generally reconstructed using a shift-and-add procedure or more complex procedures. A panoramic image depicts the anatomical structures located in a panoramic layer (a 3D mesh) to be mapped within the patient's jaw.

Generally, in x-ray panoramic imaging, two-dimensional x-ray projection images are acquired by rotating an x-ray detector and an x-ray source along a trajectory around the patient's head. Usually, the panoramic layer is pre-set for the patients to be imaged relative to the trajectory. Finally, a panoramic image is reconstructed based on the panoramic layer and the x-ray projection images.

The sharpness of the panoramic image is important for a correct diagnosis and the treatment. However, the sharpness depends significantly on the position of the panoramic layer relative to the patient's position and the patient specific dental condition.

In the prior art, the positioning of the patient is usually achieved by using a bite block and /or a head fixture. Alternatively, the position of the patient can be determined by imaging techniques using scout shots. In the prior art, the sharpness of the panoramic image can be improved by commonly known autofocus techniques in which the panoramic layer is optimized through applying local sharpness measures to obtain a sharply reconstructed panoramic image.

The above prior art techniques have the disadvantage that the patient is unnecessarily exposed with additional x-ray radiation for acquiring the scout shot information.

Furthermore, the commonly used autofocus techniques require additional computational effort for the calculation of the optimal panoramic layer.

US2020/234404A1 discloses a method for generating a panoramic image of a patient includes obtaining a digital 3D surface representation of at least a part of the patient's teeth; using the obtained digital 3D surface representation to define a customized path following the arch form of the patient's teeth; obtaining a plurality of x-ray images of at least a part of one of the patient's jaws and/or teeth; and generating the panoramic image of the patient using the customized path.

KR101146862B1 discloses a method to change from a two-dimensional panorama picture to a three-dimensional picture and the recorded media thereof.

Barone Sandro et al: "Geometrical modeling of complete dental shapes by using panoramicX-ray, digital mouth data and anatomical template", 20 January 2015, presents a methodology to create complete 3D patient dental anatomies by combiningdigital mouth models and panoramic radiographs.

### DISCLOSURE OF THE INVENTION

An objective of the present invention is to provide a system and method for generating a panoramic image in which dental condition of the patient can be mapped with improved sharpness.

This objectives is achieved by the method as defined in claim 1, and the x-ray system as defined in claim 6. The subject-matters of the dependent claims define further developments and preferred embodiments.

The method according to the invention is a computer-implemented method as set out in claim 1.

An advantageous effect of the invention is that the sharpness of the final panoramic layer can be improved by virtue of the digital 3D model of the dental condition of the patient, especially through deforming the initial panoramic layer to conform with the 3D surface of the teeth and the gingiva. The digital 3D model is used thereby as a pre-knowledge about the dental condition of the patient.

In sum, the method according to the present invention creates diagnostic and therapeutic added value for the dentist through more reliable diagnosis due to the improved sharpness of the final panoramic layer. Thus, no additional sharpness measures, e.g., autofocus, or positioning means, e.g., scout shots are necessary because by virtue of the digital 3D model, a patient-specific deformed initial panoramic layer is used for the sharpness optimization.

According to the present invention, the digital 3D model can be obtained through various methods. For example, the digital 3D model can be obtained through optically scanning of the dental condition by means of an intraoral scanner. For example, the digital 3D model can be obtained through optically scanning of an impression material by means of an intraoral scanner. For example, the digital 3D model can be obtained through using a 3D volume image which has been reconstructed from a set x-ray projection images of the patient's head. For example, the digital 3D model can be obtained through using a 3D volume image which has been reconstructed from a set of x-ray projections of an impression material of the patient's dental condition.

According to the present invention, it is also optionally considered to additionally use anatomical structures of the dental condition when finding the mapping function. In this way, impairment of the mapping function by noise can be avoided or reduced as far as possible. These anatomical structures may include one or more of the following: tooth contour; tooth tips; tooth necks; tooth crowns; approximal teeth contacts; tooth roots; tooth root tips, tooth nerve canals, tooth pulp; periodontal gap.

### BRIEF DESCRIPTION OF THE DRAWINGS

In the following description, the present invention will be explained in more detail with reference to the exemplary embodiments and with reference to the drawings, wherein.
- Fig. 1 -: shows a flow chart of a method of generating a final panoramic image of a patient according to an embodiment of the invention;
- Fig. 2 -: shows a digital 3D model of a dental condition of the patient according to an embodiment of the invention;
- Fig. 3 -: shows a schematic drawing of an x-ray system according to an embodiment of the invention;
- Fig. 4 -: shows at least some of the two-dimensional x-ray projection images acquired by rotating an x-ray detector and an x-ray source along a trajectory around the patient's head according to an embodiment of the invention;
- Fig. 5 -: shows an initial panoramic layer (3D Mesh) of the patient to be imaged relative to the trajectory according to an embodiment of the invention;
- Fig. 6 -: shows an initial panoramic image reconstructed based on the initial panoramic layer and the x-ray projection images according to an embodiment of the invention;
- Fig. 7 -: shows a plurality of reference points set on the initial panoramic image, and an anatomical structure (A) according to an embodiment of the invention;
- Fig. 8 -: shows corresponding plurality of points found on the digital 3D model ,and an anatomical structure (A) according to an embodiment of the invention;
- Fig. 9 -: shows a plurality of points selected on the initial panoramic layer which correspond to the plurality of reference points set on the initial panoramic image according to an embodiment of the invention;
- Fig. 10 -: shows a final panoramic layer obtained by deforming the initial panoramic layer in accordance with a mapping function as shown in Fig. 14 according to an embodiment of the invention;
- Fig. 11 **-**: shows a final panoramic image reconstructed based on the x-ray projection images and the final panoramic layer according to an embodiment of the invention;
- Fig. 12 -: shows a dentist acquiring a digital 3D model of a dental condition of the patient according to an embodiment of the invention;
- Fig. 13 -: shows an x-ray system having an intraoral scanner according to an embodiment of the invention;
- Fig. 14 -: shows a mapping function (F) that maps the plurality of points selected on the initial panoramic layer to the corresponding plurality of points found on the digital 3D model according to an embodiment of the invention.

The reference numbers shown in the drawings designate the elements listed below, which are referred to in the following description of the exemplary embodiments.
- 1.: X-ray system
- 2.: X-ray device
- 3.: X-ray source
- 4.: X-ray detector
- 5.: Operating unit (user interface)
- 6.: Head fixation
- 7.: Bite block
- 8.: Computing unit (Computer)
- 9.: Display device (Screen)
- 10.: Input device (Keyboard)
- 11.: Input device (Mouse)
- 12.: Intraoral Scanner
- 13.: Final panoramic image
- 14: Digital 3D model (IO Scan)
- 15.: X-ray projection images
- 16.: Initial panoramic layer (or 3D Mesh)
- 17.: Initial panoramic image
- 18.: Final panoramic layer (or deformed 3D Mesh)

### A: An anatomical structure (tooth contour)

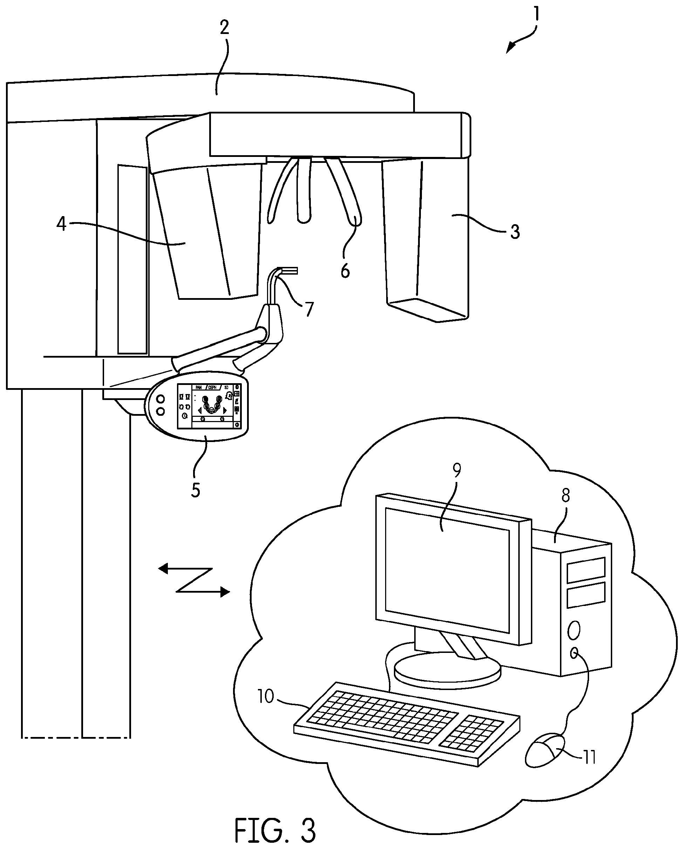

The method according to the invention, which is explained in detail below, is a computer-implemented method and can be carried out on a computer-implemented x-ray system (1) as shown in an embodiment in Fig. 3.

The present invention also includes a corresponding computer program having computer-readable code for implementing the method. The computer program is provided on a computer readable storage medium accessible to the x-ray system (1). The computerized x-ray system (1) comprises an x-ray device (2) for performing patient imaging, which generates x-ray projection images (15) or a sinogram. The x-ray device (2) has an x-ray source (3) and an x-ray detector (4) that are rotated around the patient's head during the imaging. The trajectory of the x-ray source (3) and the x-ray detector (4) during the imaging can describe a circular path. However, it can also assume a form deviating from this. If several actuators (not shown) are controlled simultaneously, a trajectory deviating from a pure circular path around the patient's head can be achieved. The patient's head can be positioned in the x-ray device (2) with a bite block (7) and optionally with a head fixation (6). The trajectory (*course*) of the x-ray source (3) and the x-ray detector (4) with respect to the bite block (7) and the head fixation (6) are known by the system (1). The x-ray detector (4) detects the x-rays emitted by the x-ray source (3) during the rotation. The x-ray projection images (15) are acquired, namely read out from the x-ray detector (4). The computerized x-ray system (1) also comprises an operating unit (5) such as a user interface, a computing unit (8) e.g., a computer which can be connected to the x-ray device (2), and a display device (9) such as a screen for visualizing any data sets resulting from the method. The computer may be connected to the x-ray device (2) via a local area network (not shown) or, alternatively, via the Internet. The computer is connected to input devices such as a keyboard (10), mouse (11), and the like. The computer may be also part of a cloud. Alternatively, the computer may be integrated into the x-ray device (2). Alternatively, all or some of the computations may take place in the cloud as cloud computing. The computer executes the computer program and provides the data sets, e.g., the panoramic images for visualization on the screen. The screen may be provided spatially separate from or integrated with the x-ray device. Preferably, the computer may also control all functions of the x-ray device (2). Alternatively, separate computers may be used for the control, operation, and panoramic image reconstruction.

The x-ray system (1) is preferably configurable as an IoT system and can be bidirectionally connected via a local area network and/or the internet (not shown) to other dental devices such as optical intraoral scanners (12), dental milling machines, additive manufacturing machines, and the like for cloud computing, data exchange, remote control, and the like.

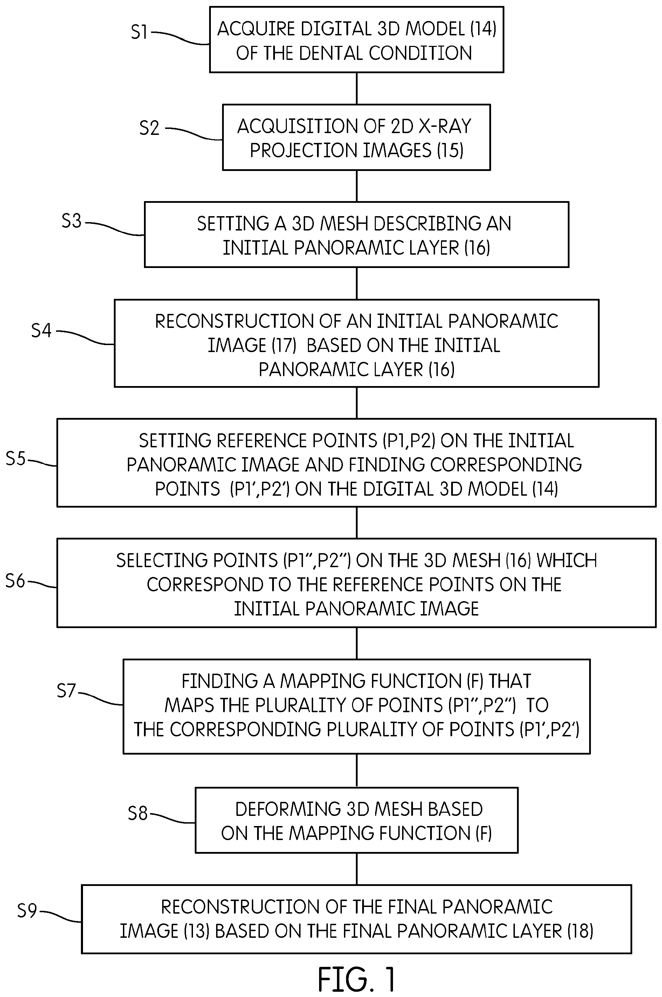

The computer implemented method is for generating a final panoramic image (13) of a patient. The method comprises the following steps (S1-S9) as shown in Fig. 1.

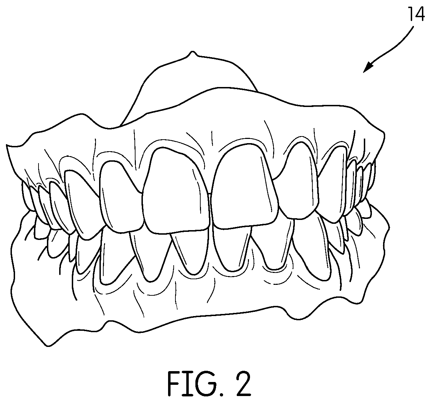

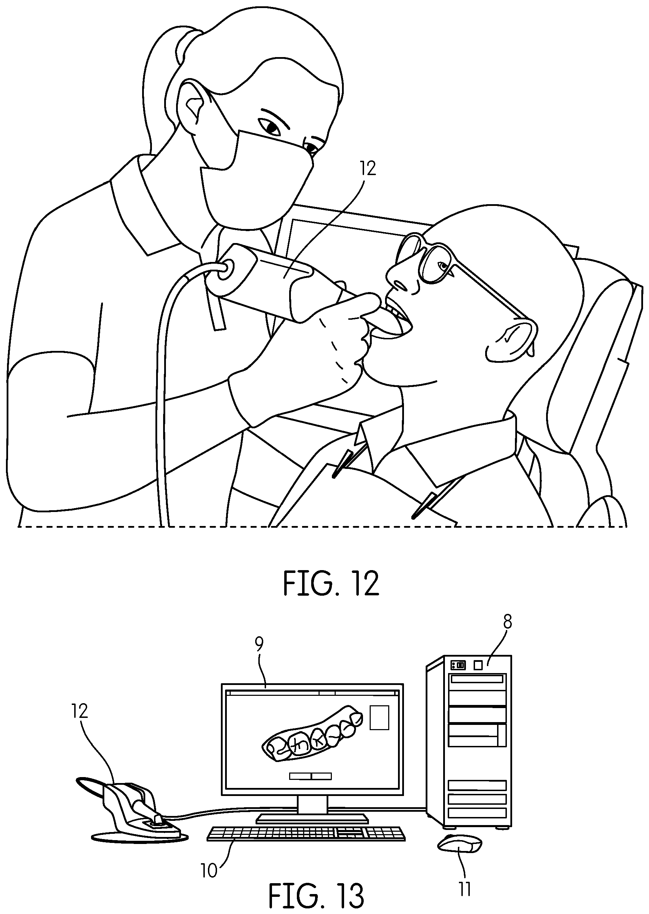

In step (S1), as shown in Fig. 2, a digital 3D model (14) of a dental condition of the patient which describes the 3D surface of the teeth, and the gingiva is acquired. The digital 3D model (14) can be obtained through one or more of the following methods, e.g, through (i) optically scanning of the dental condition by means of an intraoral scanner (12) as shown in Fig. 12 and Fig. 13, (ii) optically scanning of an impression material (not shown) by means of an intraoral scanner (12); (iii) using a 3D volume image which has been reconstructed from a set x-ray projection images of the patient's head, (iv) using a 3D volume image which has been reconstructed from a set of x-ray projections of an impression material of the patient's dental condition.

The digital 3D model (14) of the dental condition of the patient can already exist on the computing unit (8) of the dentist. Alternatively, a new digital 3D model (14) of the dental condition of the patient can be directly acquired before the x-ray panoramic imaging. Alternatively, a digital 3D model (14) of the patient can be obtained from another dentist via cloud data exchange.

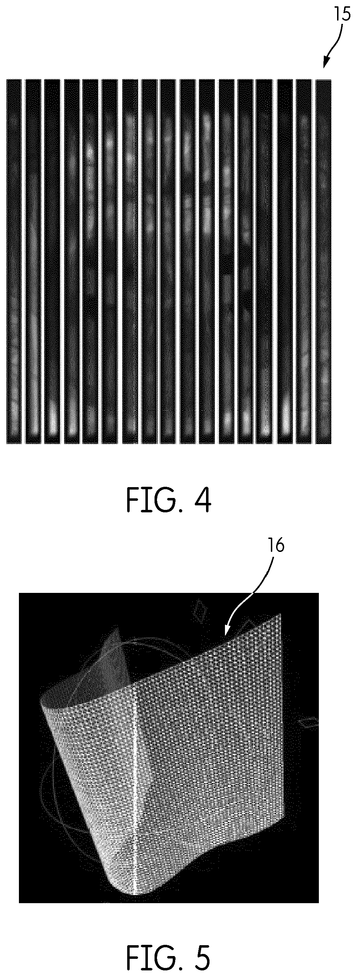

In step (S2), two-dimensional x-ray projection images (15) as shown in Fig. 4, are acquired by rotating the x-ray detector (4) and the x-ray source (3) along a trajectory around the patient's head. The system (1) has several x-ray panoramic acquisition modes for different patient anatomies. Each acquisition mode defines a specific trajectory of the x-ray detector (4) and the x-ray source (3) around the patient's head.

In step (S3), an initial panoramic layer (16) as shown in Fig. 5, of the patient to be imaged relative to the trajectory is set. The initial panoramic layer (16) describes the area to be reconstructed in the 3D space and defines the dimensions (width, height, pixel size) of initial panoramic image (17) and the anatomical structures to be mapped within the patient's jaw.

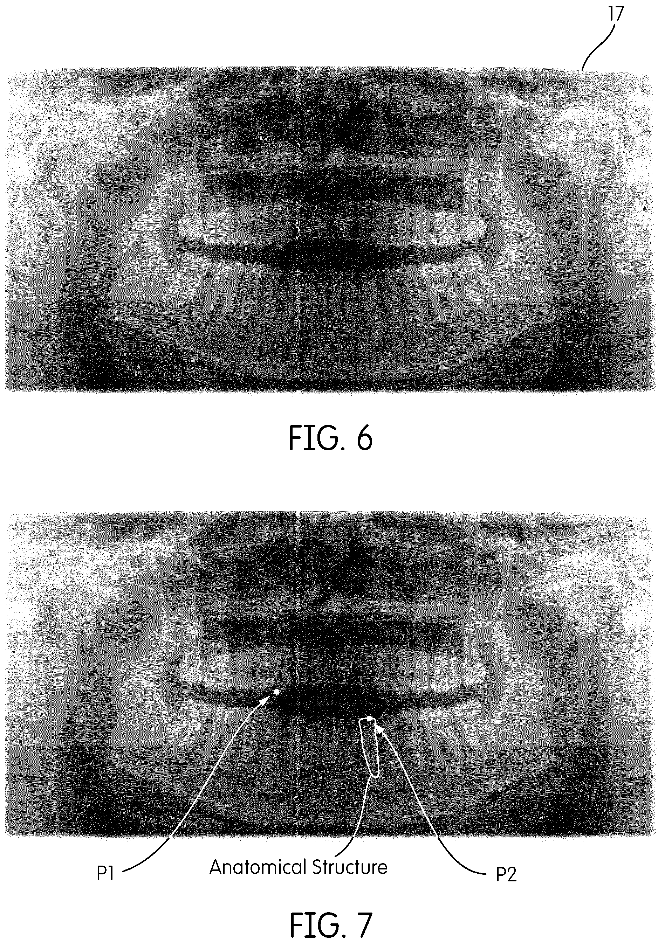

In step (S4), an initial panoramic image (17) as shown in Fig. 6, is reconstructed based on the initial panoramic layer (16) and the x-ray projection images (15). For the reconstruction of the initial panoramic image (17) a conventional Shift&Add approach may be used. All exposed x-ray projection images (15) contribute to the initial panoramic image (17). The x-ray projection images may be postprocessed, e.g., offset (dark current) and gain (beam shape, pixel based signal deviation) corrected, before reconstructing the initial panoramic image (17).

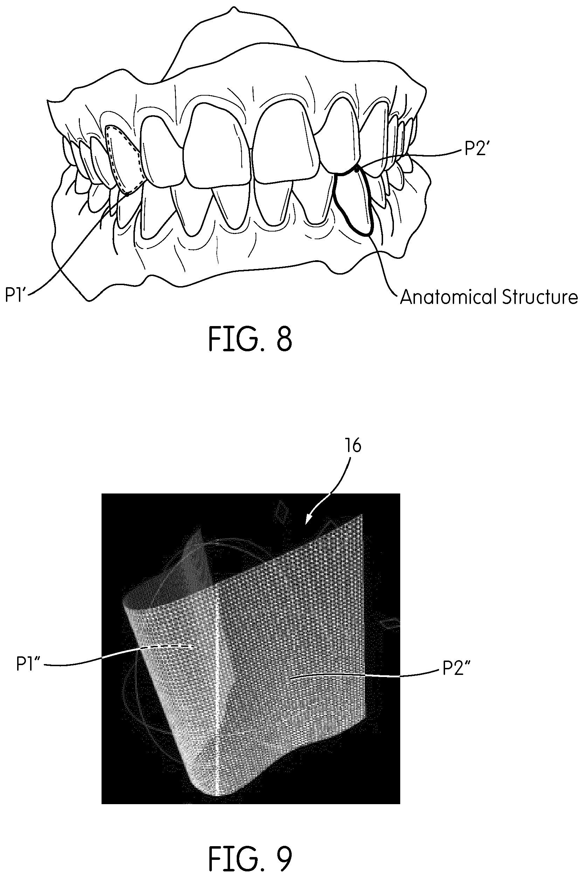

In step (S5), a plurality of reference points (P1, P2) as shown in Fig. 7, are set on the initial panoramic image (17). The number of reference points (P1, P2) may be also increased with the number of anatomical structures (A) to be used. Subsequently, the corresponding plurality of points (P1', P2') as shown in Fig. 8, are found on the digital 3D model (14). Additional image processing techniques such as pattern recognition or AI can be used to find the corresponding plurality of points (P1', P2') on the digital 3D model (14).

In step (S6), a plurality of points (P1", P2") as shown in Fig. 9, are selected on the initial panoramic layer (16) which correspond to the plurality of reference points (P1, P2) set on the initial panoramic image (17). The initial panoramic layer (16) may have the same resolution as the initial panoramic image (17). The plurality of reference (P1, P2) points define x-y positions on the initial panoramic image (17) whereas the plurality of points (P1", P2") define x-y-z coordinates on the initial panoramic layer (16) in 3D. For each reference point on the initial panoramic image (17) a corresponding point on the initial panoramic layer (16) exist.

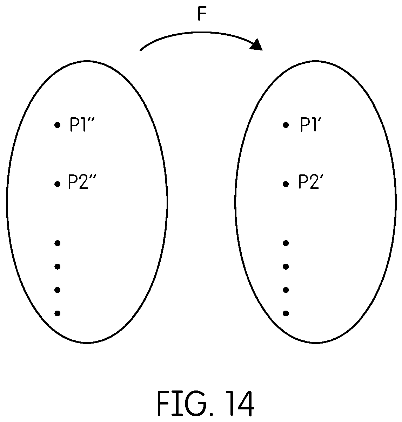

In step (S7), a mapping function (F) as shown inf Fig. 14, is found that maps the plurality of points (P1'', P2") selected on the initial panoramic layer (16) to the corresponding plurality of points (P1', P2') found on the digital 3D model (14). The mapping function (F) can be defined per pixel (point), or group of pixels (group of points). The group of pixels may each define a dental region such as separate anatomical structure. The mapping function (F) may be represented trough a transformation matrix, including rotation, translation, scaling, and/or warping.

In step (S8), the initial panoramic layer (16) is deformed in accordance with the mapping function (F) to obtain a final panoramic layer (18). Additionally, a 3D blurring filter can be applied on the final panoramic layer (18) to remove any kind of outliers before reconstruction the final panoramic image (13).

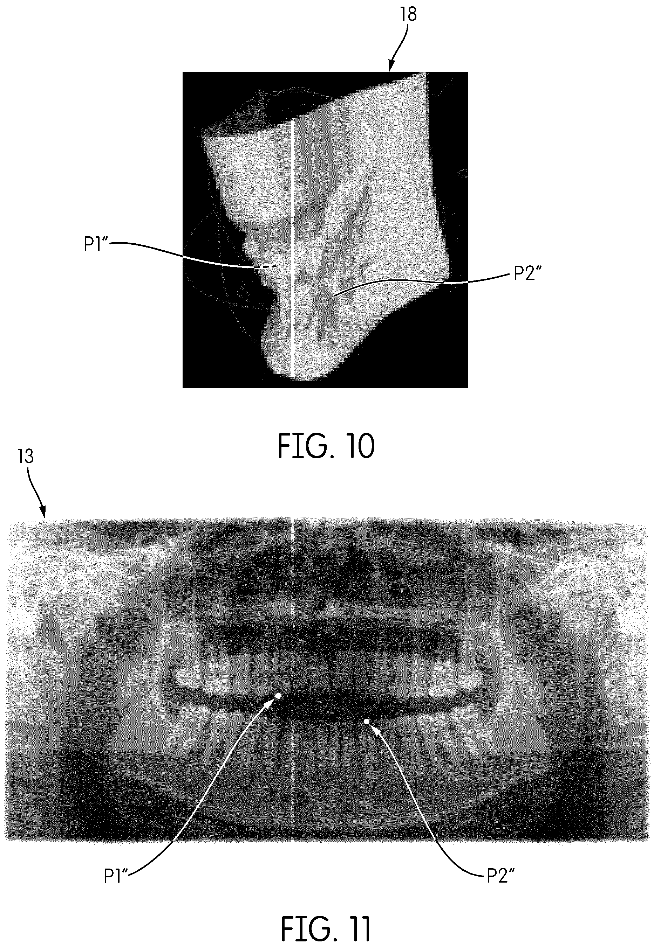

In step (S9), the final panoramic image (13) as shown in Fig. 11, is reconstructed based on the x-ray projection images (15) and the final panoramic layer (18). For the reconstruction of the final panoramic image (13) a conventional Shift&Add approach may be used. All exposed x-ray projection images (15) contribute to the initial panoramic image (17).

Finally, the final panoramic image (13) can be displayed on the display device (9). The final panoramic image (13) can be displayed on the display device (9) as a planar 2D image or as a 3D image superposed on the final panoramic layer (18).

According to a variation of the above described embodiment, it is also optionally considered to additionally use anatomical structures (A) as shown in Fig. 7, of the dental condition when finding the mapping function (F). Additional image processing techniques such as pattern recognition or AI can be used. In this embodiment, the setting step (S5) comprises the following sub steps: In a first sub step, one or more anatomical structures (A) in the initial panoramic image (17) and the same anatomical structures (A) in the digital 3D model (14) as shown in Fig.8, are determined. Subsequently, in a second sub step, the plurality of reference points are set on the anatomical structures (A) in the initial panoramic image (17), and then the corresponding plurality of points on the corresponding anatomical structures are found in the digital 3D model (14).

In this embodiment, the anatomical structures (A) may include one or more of the following: tooth contour; tooth tips; tooth necks; tooth crowns; approximal teeth contacts; tooth roots; tooth root tips; tooth nerve canals; tooth pulp; periodontal gap.

The selection of anatomical structures (A) to be used depends on the type of the digital 3D model (14) of the dental condition of the patient.

## Claims

1. A computer implemented method of generating a final panoramic image (13) of a patient, comprising the steps of:
acquiring (S1) a digital 3D model (14) of a dental condition of the patient which describes the 3D surface of the teeth and the gingiva;
acquiring (S2) two-dimensional x-ray projection images (15) by rotating an x-ray detector (4) and an x-ray source (3) along a trajectory around the patient's head;
setting (S3) an initial panoramic layer (16) of the patient to be imaged relative to the trajectory, wherein the initial panoramic layer is a 3D mesh that describes an area to be reconstructed in 3D space and defines dimensions of an initial panoramic image (17) and anatomical structures to be mapped within the patient's jaw;
reconstructing (S4) the initial panoramic image (17) based on the initial panoramic layer (16) and the x-ray projection images (15) using a Shift&Add approach;
setting (SS) a plurality of reference points (P1, P2) on the initial panoramic image (17), and finding the corresponding plurality of points (P1', P2') on the digital 3D model (14);
selecting (S6) a plurality of points (P1", P2") on the initial panoramic layer (16) which correspond to the plurality of reference points (P1, P2) set on the initial panoramic image (17);
finding (S7) a mapping function (F) that maps the plurality of points (P1", P2") selected on the initial panoramic layer (16) to the corresponding plurality of points (P1', P2') found on the digital 3D model (14);
deforming (S8) the initial panoramic layer (16) in accordance with the mapping function (F) to obtain a final panoramic layer (18);
reconstructing (S9) the final panoramic image (13) based on the x-ray projection images (15) and the final panoramic layer (18);
displaying the final panoramic image (13).

2. The method according to claim 1, **characterized in that**
in the acquiring step (S1) the digital 3D model (14) can be obtained through one or more of the following methods:
- optically scanning of the dental condition by means of an intraoral scanner (12);
- optically scanning of an impression material by means of an intraoral scanner (12); or
- using a 3D volume image which has been reconstructed from a set x-ray projection images of the patient's head;
- using a 3D volume image which has been reconstructed from a set ofx-ray projection images of an impression material of the patient's dental condition.

3. The method according to claim 1 or 2, **characterized in that** in the setting step (SS) comprises the subs steps of:
determining one or more anatomical structures (A) in the initial panoramic image (17) and the same anatomical structures (A) in the digital 3D model (14);
setting the plurality of reference points on the anatomical structures in the initial panoramic image (17), and finding the corresponding plurality of points on the corresponding anatomical structures in the digital 3D model (14).

4. The method according to claim 3, **characterized in that** the anatomical structures (A) include one or more of the following:
tooth contour;
tooth tips;
tooth necks;
tooth crowns;
approximal teeth contacts;
tooth roots;
tooth root tips
tooth nerve canals
tooth pulp;
periodontal gap.

5. A computer program comprising computer-readable code which, when executed by a computer-assisted x-ray system (1), causes the system (1) to perform the method of any one of the preceding claims.

6. A computer-assisted x-ray system (1) comprising a computing unit (8) configured to execute the computer program according to claim 5.

## Patentansprüche

1. Computerimplementiertes Verfahren zum Erzeugen eines panoramischen Endbildes (13) eines Patienten, umfassend die folgenden Schritte:
Erfassen (S1) eines digitalen 3D-Modells (14) eines Zahnzustands des Patienten, das die 3D-Oberfläche der Zähne und der Gingiva beschreibt;
Erfassen (S2) zweidimensionaler Röntgenprojektionsbilder (15) durch Drehen eines Röntgendetektors (4) und einer Röntgenquelle (3) entlang einer Trajektorie um den Kopf des Patienten;
Einstellen (S3) einer anfänglichen Panoramaschicht (16) des abzubildenden Patienten relativ zur Trajektorie, wobei die anfängliche Panoramaschicht ein 3D-Netz ist, das einen zu rekonstruierenden Bereich im 3D-Raum beschreibt und Dimensionen eines panoramischen Anfangsbilds (17) und zuzuordnende anatomische Strukturen innerhalb des Kiefers des Patienten definiert;
Rekonstruieren (S4) des panoramischen Anfangsbilds (17) basierend auf der anfänglichen Panoramaschicht (16) und den Röntgenprojektionsbildern (15) unter Verwendung eines Shift&Add-Ansatzes;
Einstellen (SS) einer Vielzahl von Referenzpunkten (P1, P2) auf dem panoramischen Anfangsbild (17) und Finden der entsprechenden Vielzahl von Punkten (Pl', P2') auf dem digitalen 3D-Modell (14);
Auswählen (S6) einer Vielzahl von Punkten (P1", P2") auf der anfänglichen Panoramaschicht (16), die der Vielzahl von Referenzpunkten (P1, P2) entsprechen, die auf dem panoramischen Anfangsbild (17) eingestellt sind;
Finden (S7) einer Zuordnungsfunktion (F), die die Vielzahl von Punkten (P1", P2"), die auf der anfänglichen Panoramaschicht (16) ausgewählt sind, der entsprechenden Vielzahl von Punkten (P1', P2') zuordnet, die auf dem digitalen 3D-Modell (14) gefunden werden;
Verformen (S8) der anfänglichen Panoramaschicht (16) gemäß der Zuordnungsfunktion (F), um eine endgültige Panoramaschicht (18) zu erlangen;
Rekonstruieren (S9) des panoramischen Endbilds (13) basierend auf den Röntgenprojektionsbildern (15) und der endgültigen Panoramaschicht (18);
Anzeige des panoramischen Endbildes (13).

2. Verfahren nach Anspruch 1, **dadurch gekennzeichnet, dass**
im Erfassungsschritt (S1) das digitale 3D-Modell (14) durch eines oder mehrere der folgenden Verfahren erlangt werden kann:
- optisches Abtasten des Zahnzustandes mittels eines Intraoralscanners (12);
- optisches Abtasten eines Abdruckmaterials mittels eines Intraoralscanners (12); oder
- Verwenden eines 3D-Volumenbildes, das aus einem Satz von Röntgenprojektionsbildern des Kopfes des Patienten rekonstruiert wurde;
- Verwenden eines 3D-Volumenbilds, das aus einem Satz von Röntgenprojektionsbildern eines Abdruckmaterials des Zahnzustands des Patienten rekonstruiert wurde.

3. Verfahren nach Anspruch 1 oder 2, **dadurch gekennzeichnet, dass** der Einstellschritt (SS) die folgenden Unterschritte umfasst:
Bestimmen einer oder mehrerer anatomischer Strukturen (A) in dem panoramischen Anfangsbild (17) und der gleichen anatomischen Strukturen (A) in dem digitalen 3D-Modell (14);
Einstellen der Vielzahl von Referenzpunkten auf den anatomischen Strukturen in dem panoramischen Anfangsbild (17) und Finden der entsprechenden Vielzahl von Punkten auf den entsprechenden anatomischen Strukturen in dem digitalen 3D-Modell (14).

4. Verfahren nach Anspruch 3, **dadurch gekennzeichnet, dass** die anatomischen Strukturen (A) eines oder mehrere der Folgenden enthalten:
Zahnkontur;
Zahnspitzen;
Zahnhälse;
Zahnkronen;
Kontakte aneinandergrenzender Zähne;
Zahnwurzeln;
Zahnwurzelspitzen
Zahnnervenkanäle
Zahnpulpa;
Periodontalspalt.

5. Computerprogramm, umfassend computerlesbaren Code, der, wenn er durch ein computergestütztes Röntgensystem (1) ausgeführt wird, das System (1) veranlasst, das Verfahren nach einem der vorhergehenden Ansprüche durchzuführen.

6. Computergestütztes Röntgensystem (1), umfassend eine Recheneinheit (8), die zum Ausführen des Computerprogramms nach Anspruch 5 konfiguriert ist.

## Revendications

1. Procédé mis en œuvre par ordinateur de génération d'une image panoramique finale (13) d'un patient, comprenant les étapes de :
acquisition (S1) d'un modèle numérique 3D (14) d'un état dentaire du patient qui décrit la surface 3D des dents et de la gencive ;
acquisition (S2) d'images de projection de rayons X bidimensionnelles (15) en faisant tourner un détecteur de rayons X (4) et une source de rayons X (3) le long d'une trajectoire autour de la tête du patient ;
réglage (S3) d'une couche panoramique initiale (16) du patient devant être imagée par rapport à la trajectoire, ladite couche panoramique initiale étant un maillage 3D qui décrit une zone devant être reconstruite dans l'espace 3D et définit les dimensions d'une image panoramique initiale (17) et les structures anatomiques devant être cartographiées au sein de la mâchoire du patient ;
reconstruction (S4) de l'image panoramique initiale (17) sur la base de la couche panoramique initiale (16) et des images de projection de rayons X (15) à l'aide d'une approche Shift&Add ;
définition (SS) d'une pluralité de points de référence (P1, P2) sur l'image panoramique initiale (17), et recherche de la pluralité de points correspondante (P1', P2') sur le modèle numérique 3D (14) ;
sélection (S6) d'une pluralité de points (P1", P2") sur la couche panoramique initiale (16) qui correspondent à la pluralité de points de référence (P1, P2) définis sur l'image panoramique initiale (17) ;
recherche (S7) d'une fonction de mappage (F) qui mappe la pluralité de points (P1", P2") sélectionnés sur la couche panoramique initiale (16) à la pluralité de points correspondante (P1', P2') trouvée sur le modèle numérique 3D (14) ;
déformation (S8) de la couche panoramique initiale (16) conformément à la fonction de mappage (F) pour obtenir une couche panoramique finale (18) ;
reconstruction (S9) de l'image panoramique finale (13) sur la base des images de projection de rayons X (15) et de la couche panoramique finale (18) ;
affichage de l'image panoramique finale (13).

2. Procédé selon la revendication 1, **caractérisé en ce que**
à l'étape d'acquisition (S1), le modèle numérique 3D (14) peut être obtenu par un ou plusieurs des procédés suivants :
- balayage optique de l'état dentaire au moyen d'un scanner intra-buccal (12) ;
- balayage optique d'un matériau d'impression au moyen d'un scanner intra-buccal (12) ; ou
- utilisation d'une image de volume 3D qui a été reconstruite à partir d'un ensemble d'images de projection de rayons X de la tête du patient ;
- utilisation d'une image de volume 3D qui a été reconstruite à partir d'un ensemble d'images de projection de rayons X d'un matériau d'impression de l'état dentaire du patient.

3. Procédé selon la revendication 1 ou 2, **caractérisé en ce que** l'étape de réglage (SS) comprend les sous-étapes de :
détermination d'une ou de plusieurs structures anatomiques (A) dans l'image panoramique initiale (17) et des mêmes structures anatomiques (A) dans le modèle numérique 3D (14) ;
définition de la pluralité de points de référence sur les structures anatomiques dans l'image panoramique initiale (17), et recherche de la pluralité de points correspondante sur les structures anatomiques correspondantes dans le modèle numérique 3D (14).

4. Procédé selon la revendication 3, **caractérisé en ce que** les structures anatomiques (A) comprennent un ou plusieurs des éléments suivants :
le contour des dents ;
des pointes de dents ;
des collets de dents ;
des couronnes dentaires ;
des contacts approximatifs de dents ;
des racines de dents ;
des pointes de racines de dents
des canaux nerveux de dents
la pulpe dentaire ;
l'intervalle parodontal.

5. Programme informatique comprenant un code lisible par ordinateur qui, lorsqu'il est exécuté par un système à rayons X assisté par ordinateur (1), amène le système (1) à exécuter le procédé de l'une quelconque des revendications précédentes.

6. Système de radiographie assisté par ordinateur (1) comprenant une unité informatique (8) configurée pour exécuter le programme informatique selon la revendication 5.
